(19) Europäisches Patentamt
European Patent Office
Office européen des brevets

(11) **EP 3 169 983 B1**

(12) **FASCICULE DE BREVET EUROPEEN**

(45) Date de publication et mention
de la délivrance du brevet:
**19.02.2020 Bulletin 2020/08**

(51) Int Cl.:
***G01L 1/14*** *(2006.01)*     ***A61B 5/103*** *(2006.01)*
***G01L 5/16*** *(2020.01)*

(21) Numéro de dépôt: **15753106.2**

(22) Date de dépôt: **16.07.2015**

(86) Numéro de dépôt international:
**PCT/FR2015/051947**

(87) Numéro de publication internationale:
**WO 2016/009151 (21.01.2016 Gazette 2016/03)**

(54) **SYSTÈME A RÉSEAU DE CELLULES DE CAPTEURS CAPACITIFS DE PRESSION ET DE CISAILLEMENT ET PROCÉDÉ DE FABRICATION**

SYSTEM MIT EINEM ZELLULAREN NETZWERK AUS KAPAZITIVEN DRUCK- UND SCHERBELASTUNGSSENSOREN UND HERSTELLUNGSVERFAHREN

SYSTEM COMPRISING A CELLULAR NETWORK OF CAPACITIVE PRESSURE AND SHEAR-STRESS SENSORS AND MANUFACTURING PROCESS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.07.2014 FR 1456955**

(43) Date de publication de la demande:
**24.05.2017 Bulletin 2017/21**

(73) Titulaire: **Feetme**
**78000 Versailles (FR)**

(72) Inventeurs:
• **MATHIEU, Alexis**
**F-86210 Bonneuil-Matours (FR)**
• **MERCIER, Julien**
**F-18000 Bourges (FR)**

(74) Mandataire: **Jacobacci Coralis Harle**
**32, rue de l'Arcade**
**75008 Paris (FR)**

(56) Documents cités:
FR-A1- 2 878 956     US-A1- 2006 267 140
US-A1- 2014 076 066

EP 3 169 983 B1

**Description**

**Domaine technique**

La présente invention se rapporte à un système à réseau de capteurs pour mesurer des forces de pression avec une haute résolution spatiale. Une force de pression se décompose généralement, d'une part, en une force de compression qui s'applique perpendiculairement à la surface du capteur de pression, et, d'autre part, en une force de cisaillement qui s'applique suivant une direction oblique par rapport à la surface du capteur de pression.

Plus précisément, l'invention concerne une semelle à capteurs de pression destinée à mesurer des forces de compression et de cisaillement avec une haute résolution spatiale.

**Arrière-plan technologique**

Dans le domaine médical ou le domaine sportif, il est souhaitable de connaître la répartition des forces de pression exercées par les pieds d'une personne, en position statique ou dynamique. Dans le domaine médical, une semelle à capteurs de pression trouve des applications comme semelle de diagnostic en podologie ou en orthopédie. Portée au quotidien par un patient diabétique atteint de neuropathie, une semelle à capteurs de pression peut permettre d'améliorer la prévention de lésions au pied. Dans le domaine sportif, une semelle à capteurs de pression portée par un sportif et connectée à un téléphone intelligent permet au sportif de quantifier sa course. L'analyse de la répartition des forces de pression s'appliquant en particulier au niveau de la plante du pied pendant la marche, la course ou le saut peut permettre au sportif de corriger consciemment un déséquilibre postural afin d'éviter l'apparition de douleurs ou de blessures.

Il existe des dispositifs à capteurs de pression basés sur des technologies optiques, magnétiques ou électriques et en particulier à capteurs résistifs, à capteurs inductifs (voir EP 20607876) ou à capteurs capacitifs (voir US 7 343 813, US 2014/076066, US2006/247140, FR2878956).

Un avantage des capteurs capacitifs de pression est d'être peu sensibles aux variations de température.

Un capteur capacitif de pression comporte au moins deux électrodes séparées par un matériau diélectrique. La capacité électrique d'un capteur capacitif est donnée par la formule d'un condensateur entre deux plaques :

$$C = \frac{\varepsilon.S}{L} \qquad\qquad (\mathrm{I})$$

où C représente la capacité électrique du capteur capacitif, de type condensateur, S la surface des électrodes mises en regard, $L$ la distance entre les deux électrodes et $\varepsilon$ la constante diélectrique du matériau entre les électrodes.

Sous l'effet d'une force de pression normale, la variation d'épaisseur $L$ du matériau diélectrique produit une variation inversement proportionnelle de la capacité électrique $C$ du capteur.

On connaît par exemple du document US 5449002 un capteur capacitif de pression basé sur un diélectrique de polyuréthane résilient pris en sandwich entre deux conducteurs électriques. La variation de la capacité électrique de ce capteur est quasiment linéaire en fonction du poids appliqué, ce qui permet une détection aisée. Ce capteur peut être utilisé sous forme de semelle de chaussure, de poignée de préhension ou de support pour mesurer les forces de compression dans différents appareils médicaux tels que béquille, fauteuil roulant, tapis de marche. Cependant, un tel capteur ne fournit pas des mesures de pression résolues spatialement sur la surface du capteur. De plus, ce capteur capacitif de pression ne permet pas de discriminer une pression normale et une pression induite par une force de cisaillement.

Or, par application de la formule (I), sous l'effet d'une force de cisaillement, une variation de la surface S des électrodes en regard, produit une variation proportionnelle de la capacité électrique du capteur. Dans le cas d'un matériau diélectrique élastiquement déformable, la variation de surface S induite par la force de cisaillement s'accompagne généralement d'une variation de l'épaisseur $L$. Il est donc nécessaire de mesurer indépendamment la variation d'épaisseur pour extraire de la mesure de variation de capacité, une mesure de la variation de surface S, afin d'en déduire une mesure de la force de cisaillement.

Récemment, différents exemples de multi-capteurs capacitifs ont permis de discriminer la mesure de pression normale de la mesure de force de cisaillement (voir US 2013/0093437, US 8250926). Les capteurs capacitifs de pression et de cisaillement sont utilisés notamment dans le domaine des écrans tactiles, des interfaces haptiques, des textiles intégrant des capteurs.

Cependant, l'intégration d'un grand nombre de capteurs capacitifs de pression pour fabriquer un réseau de capteurs à haute résolution spatiale, a pour inconvénient de nécessiter un nombre encore plus grand de connections électriques reliant le réseau de capteurs au système de mesure.

Le document R. Supraneni, Q. Guo, Y. Xie, D.J. Young and C.H. Mastrangelo « A three-axis high-resolution capacitive tactile imager system based on floating comb electrodes », Journal of Micromechanis and Microengineering, 23 (2013) 075004, décrit le design et la fabrication d'un imageur tactile à haute résolution spatiale pour la mesure des forces de compression et de cisaillement. L'imageur tactile comporte un diélectrique formé d'une feuille de polymère siliconé et un circuit imprimé flexible (FPCB). Chaque cellule de l'imageur tactile comporte deux capacités sensibles à des déplacements suivant une direction X et deux capacités sensibles à des déplacements suivant une direction Y. Les quatre capacités d'une cellule comportent, sur une face du diélectrique, des électrodes flottantes, et, sur l'autre face du diélectrique, un FPCB supportant des électrodes, en forme de peigne, reliées à deux pistes électriques verticales et deux pistes électriques horizontales. La mesure électrique des quatre capacités d'une cellule requiert l'adressage multiplexé des pistes électriques verticales et horizontales, pour fournir des mesures de pression normale, suivant la direction Z, et de cisaillement, suivant les directions X et Y. Néanmoins, les pistes électriques horizontales et verticales étant déposées sur un même circuit imprimé, le procédé de fabrication du circuit imprimé requiert la superposition d'au moins deux niveaux de pistes électriques reliés par des interconnections ou vias. Un inconvénient du procédé de fabrication de circuit imprimé à double niveau est de requérir un plus grand nombre d'étapes de fabrication. De plus, ce procédé nécessite un substrat de circuit imprimé rigide, qui peut convenir pour des applications tactiles, mais ne convient généralement pas pour une application à une semelle à capteurs de pression. Enfin, les interconnections entre deux niveaux d'électrodes déposées sur un substrat déformable sont fragiles et peuvent créer des défauts électriques.

Contrairement aux applications tactiles où le substrat est généralement rigide et peut être épais, une semelle à capteurs de pression doit présenter à la fois une faible épaisseur, inférieure à quelques millimètres, et une très grande flexibilité. Une semelle à capteurs de pression doit supporter une dynamique de pression allant de 0 à 15 kg/cm$^2$.

D'autre part, un inconvénient des capteurs capacitifs à base de matériaux diélectriques déformables est que leur déformation présente généralement de l'hystérésis, susceptible d'induire des erreurs de mesure.

De plus, les capteurs capacitifs de force de cisaillement présentent une sensibilité inférieure aux capteurs de pression normale. Un capteur capacitif de force de cisaillement doit en général s'étendre sur une surface plus étendue qu'un capteur capacitif de pression normale.

Il existe donc un besoin d'un système et d'une méthode permettant de fabriquer un système de réseau de capteurs pour mesurer des forces de pression avec une haute résolution spatiale, tout en ayant une faible épaisseur et une grande flexibilité, notamment pour l'application à une semelle à capteurs de pression.

Un des buts de l'invention est de proposer un système à réseau de capteurs pour mesurer des forces de pression avec une haute résolution spatiale, présentant une faible épaisseur et un nombre de connections électriques limité. Un autre but de l'invention est de fournir non seulement des mesures de force de compression, mais aussi des mesures de forces de cisaillement.

Un autre but de l'invention est de proposer un système à réseau de capteurs qui convient pour des semelles dans le domaine médical, sportif ou récréatif.

## Objet de l'invention

La présente invention a pour but de remédier aux inconvénients des techniques antérieures et concerne un système à réseau de capteurs selon la revendication 1.

Le système à réseau de capteurs permet ainsi de mesurer avec une haute résolution spatiale la répartition spatiale des vecteurs force appliqués au réseau de capteur. L'architecture du système permet de fournir des mesures d'un grand nombre de capteurs via un nombre de connections électriques extrêmement réduit.

Les avantages de ce système à réseau de capteurs sont sa faible épaisseur, la densité spatiale des mesures ainsi que la capacité du système à mesurer des forces de frottement.

De plus, les capteurs capacitifs sont peu sensibles aux variations de température et essentiellement sensibles aux effets mécaniques.

Dans un mode de réalisation particulier et avantageux, la feuille de matériau diélectrique élastiquement déformable en compression et en cisaillement est en un matériau choisi parmi : une matière naturelle telle qu'un liège, ou un élastomère d'origine naturelle, comme par exemple un caoutchouc ou en élastomère synthétique, en particulier un uréthane, un silicone, un butyl rubber, un polymère, un néoprène, un polyuréthane ou un polyisoprène. De façon avantageuse, la feuille de matériau diélectrique est sous forme de mousse (par exemple mousse d'élastomère, en particulier mousse d'uréthane) ou de matériau micro-architecturé (par exemple du liège).

Dans un mode de réalisation particulier, ladite première électrode et ladite première piste électriquement conductrice sont imprimées sur une feuille en matériau électriquement isolant et flexible, et, respectivement, ladite deuxième électrode et lesdites autres pistes électriquement conductrices sont imprimées sur une autre feuille en matériau électriquement isolant et flexible.

Selon un mode de réalisation préféré, la première piste électriquement conductrice et la deuxième piste électriquement conductrice d'une cellule sont reliées à un système électronique adapté pour mesurer une variation de la capacité électrique du capteur capacitif de pression normale, le système électronique étant adapté pour en déduire une force de pression normale appliquée sur ledit capteur capacitif de pression normale le long de la première direction.

Au moins une cellule du réseau de capteurs comprend un deuxième capteur capacitif de cisaillement suivant une deuxième direction et un troisième capteur capacitif de cisaillement suivant une troisième direction, chaque capteur capacitif de cisaillement étant constitué d'une première électrode fixée sur la première face de la feuille de matériau diélectrique et d'une deuxième électrode fixée sur la deuxième face de la feuille de matériau diélectrique, lesdites première et deuxième électrodes des capteurs capacitifs de cisaillement étant en forme de peigne, lesdites premières électrodes des capteurs capacitifs d'une cellule étant reliées en série, la deuxième électrode du capteur capacitif de cisaillement suivant la deuxième direction étant reliée à une troisième piste électriquement conductrice reliant une ligne de capteurs capacitifs de cisaillement suivant la deuxième direction du réseau de capteurs ; et la deuxième électrode du capteur capacitif de cisaillement suivant la troisième direction étant reliée à une quatrième piste électriquement conductrice reliant une colonne de capteurs capacitifs de cisaillement suivant la troisième direction du réseau de capteurs.

De manière complémentaire, la première piste électriquement conductrice et la troisième piste électriquement conductrice d'une cellule sont reliées audit système électronique, qui est adapté pour mesurer une variation de la capacité électrique du deuxième capteur capacitif de force de cisaillement suivant la deuxième direction, le système électronique étant adapté pour en déduire l'amplitude et le sens d'une force de cisaillement appliquée sur ledit capteur capacitif de force de cisaillement le long de la deuxième direction.

De façon avantageuse, la première piste électriquement conductrice et la quatrième piste électriquement conductrice d'une cellule sont reliées audit système électronique, qui est adapté pour mesurer une variation de la capacité électrique du troisième capteur capacitif de cisaillement suivant la troisième direction, le système électronique étant adapté pour en déduire l'amplitude et le sens d'une force de cisaillement appliquée sur ledit troisième capteur capacitif de cisaillement le long de la troisième direction.

Dans un mode de réalisation particulièrement avantageux, lesdites pistes électriquement conductrices sont reliées à des moyens de mesure d'une variation de la capacité électrique de capteur capacitif par des liaisons filaires ou sans fil.

Avantageusement, le système à réseau de capteurs de pression comprend un dispositif d'affichage des mesures de force de pression de cisaillement dans lequel le dispositif d'affichage est configuré pour représenter graphiquement, en fonction de la disposition du réseau de capteur, la pression normale mesurée par chaque cellule du réseau de capteur et simultanément l'amplitude et la direction de la force de cisaillement mesurée par chaque cellule du réseau de capteur.

L'invention trouvera une application particulièrement intéressante dans la fabrication d'une semelle pour chaussure comprenant un système à réseau de capteurs de pression selon l'un des modes de réalisation décrits.

## Description détaillée d'un exemple de réalisation

La présente invention concerne également les caractéristiques qui ressortiront au cours de la description qui va suivre et qui devront être considérées isolément ou selon toutes leurs combinaisons techniquement possibles.

Cette description donnée à titre d'exemple non limitatif fera mieux comprendre comment l'invention peut être réalisée en référence aux dessins annexés sur lesquels :

- la figure 1 représente schématiquement une vue de dessus d'une cellule de capteurs capacitifs de pression selon un mode de réalisation de l'invention ;
- la figure 2 représente schématiquement une vue en coupe suivant la ligne AA de la cellule de capteurs capacitifs de pression de la figure 1 ;
- la figure 3 illustre la cellule de capteurs capacitifs de pression de la figure 2 soumis à une force de cisaillement appliquée dans la direction de l'axe X ;
- la figure 4 représente schématiquement une vue de dessus d'un capteur capacitif de pression selon une variante de l'invention ;
- la figure 5 représente une mesure de force de pression normale en appliquant une force de pression croissante puis décroissante, et illustre l'effet d'hystérésis du capteur ;
- la figure 6 illustre une vue de dessus d'un réseau de capteurs capacitifs de pression normale et de force de cisaillement ;
- les figures 7-8 représentent schématiquement une vue de dessus des électrodes d'un réseau matriciel de capteurs capacitifs de pression normale, la figure 7 correspondant aux électrodes fixées sur une face du diélectrique et la figure 8 correspondant aux électrodes fixées sur l'autre face du diélectrique ;
- la figure 9 représente schématiquement une vue de dessus d'une semelle de chaussure comprenant un réseau de capteurs capacitifs de pression ;
- la figure 10 illustre une représentation graphique de mesures de pression normale et de force de cisaillement obtenu avec une semelle à capteurs capacitifs de pression telle que représentée sur la figure 9 ;

- la figure 11 illustre une autre représentation graphique de mesures de pression normale et de force de cisaillement obtenu avec une semelle à capteurs capacitifs de pression ;
- la figure 12 illustre une étape d'un procédé de fabrication d'une semelle à réseau de cellules de capteurs capacitifs de pression suite à l'impression par sérigraphie d'un premier motif d'électrodes ;
- la figure 13 illustre une autre étape d'un procédé de fabrication d'une semelle à réseau de cellules de capteurs capacitifs de pression suite à l'impression par sérigraphie d'un deuxième motif d'électrodes.

Dispositif

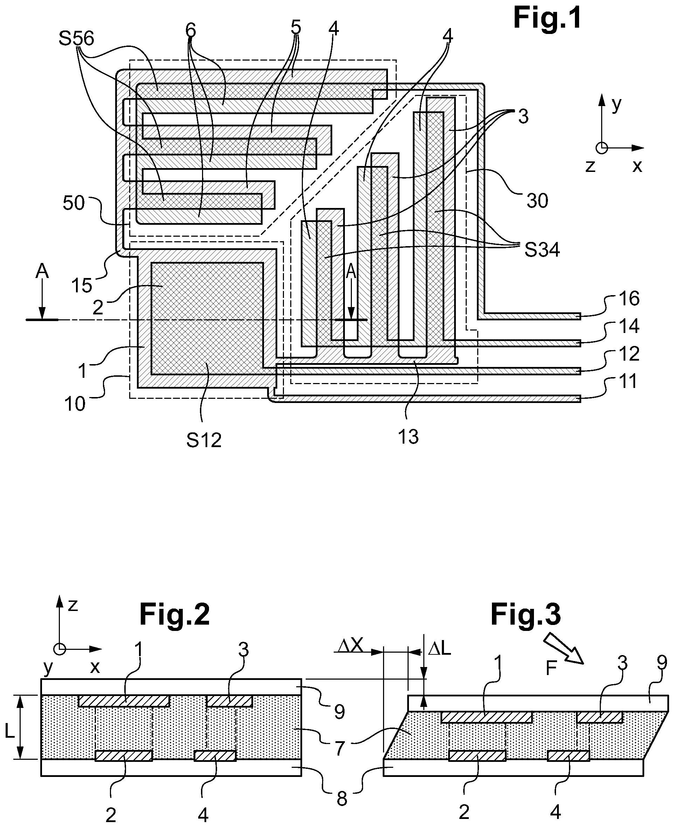

Sur la figure 1, on a représenté, en vue de dessus, une cellule de capteurs capacitifs de pression selon un mode de réalisation de l'invention. Plus précisément, la figure 1 représente en superposition le dessin des électrodes d'une cellule 20 de capteurs capacitifs. Les électrodes sont disposées sur les faces opposées d'une feuille d'un matériau diélectrique élastiquement déformable en compression et en cisaillement. On a aussi représenté un repère orthonormé XYZ, l'axe Z étant perpendiculaire au plan de la figure 1.

Sur les figures 2-3, on a représenté une vue en coupe partielle suivant la ligne AA de la cellule de capteurs capacitifs de la figure 1. L'axe Y du repère orthonormé XYZ est perpendiculaire au plan des figures 2-3. Sur la figure 2, la cellule 20 est au repos. Sur la figure 3 on a représenté la cellule 20 sous l'effet d'une force F ayant, de manière générale, une composante de suivant l'axe Z, aussi appelée force normale, et deux composantes suivant les axes X et Y, aussi appelées forces de cisaillement.

La cellule 20 comporte un capteur capacitif 10 de pression normale suivant la direction Z, un capteur capacitif de cisaillement 30 suivant la direction X et un autre capteur capacitif de cisaillement 50 suivant la direction Y.

La cellule de capteurs capacitifs comprend une feuille de matériau diélectrique 7 élastiquement déformable en compression et en cisaillement. De façon avantageuse, la feuille de matériau diélectrique 7 est formée d'une feuille de matériau élastomère. Parmi les matériaux élastomères, on cite en particulier un élastomère d'origine naturelle, le caoutchouc, et des élastomères synthétiques, les silicones. On définit la résilience mécanique d'un matériau élastomère comme étant le rapport, souvent exprimé en %, de l'énergie restituée après déformation, à l'énergie fournie pour déformer l'élastomère sous chargement cyclique. L'hystérésis correspondant au taux d'énergie dissipée, l'hystérésis est le complément de la résilience mécanique. Une grande résilience correspond à une faible hystérésis. La plupart des matériaux élastomères ont une résilience mécanique assez élevée. Toutefois, l'élastomère qui présente la résilience la plus élevée, donc l'hystérésis la plus faible, est le caoutchouc naturel. De plus, le caoutchouc naturel est bon marché et possède une bonne résistance à l'abrasion. On observe aussi une très faible hystérésis des silicones mous purs, c'est-à-dire non renforcés de particules.

Par exemple la feuille de matériau diélectrique 7 est formée d'une feuille de caoutchouc naturel, de silicone ou d'uréthane. Par exemple, la feuille de matériau diélectrique 7 se présente sous la forme d'une mousse à cellules fermées. De préférence, la feuille de matériau diélectrique 7 présente un module d'Young compris entre 1 et 5 MPa adapté pour les mesures de compression.

De façon avantageuse, pour les mesures de cisaillement, la feuille de matériau diélectrique 7 a un module de Poisson, définissant sa compressibilité, compris entre 0 et 0,5 et de préférence inférieur à 0,1. La détermination de la valeur du module de Poisson résulte d'un compromis : une valeur de 0,5 est idéale pour une mesure de cisaillement mais dans ce cas, le matériau étant incompressible, la sensibilité en pression normale est très faible. Une valeur intermédiaire du module de Poisson autour de 0,2 permet une bonne sensibilité à la fois en mesure de pression normale et en mesure de force de cisaillement. Parmi les matériaux ayant un module de Poisson de l'ordre de 0,5 on trouve notamment les matériaux suivants : le butyl rubber, les mousses polymère, le néoprène, les silicones, le polyuréthane et le polyisoprène. Pour obtenir un module de Poisson inférieur à 0,5 on s'oriente vers les matériaux micro-architecturés. En particulier, il existe un matériau qui présente à l'état naturel un module de Poisson nul (V=0) : le liège. Les mousses peuvent aussi avoir un module de Poisson compris entre 0,1 et 0,4. Cependant la plupart des mousses se tassent en compression.

Avantageusement, le matériau diélectrique a une constante diélectrique comprise entre 3 et 10 kV/mm.

De préférence, l'épaisseur de la feuille de matériau diélectrique 7 est comprise entre 0,2 et 1mm.

Dans l'exemple de réalisation illustré sur la figure 1, la cellule 20 fait environ 1cm de côté. La cellule 20 comporte des électrodes 1, 2, 3, 4 disposées sur les faces opposées de feuille de matériau diélectrique 7 pour former des capteurs capacitifs. Des pistes électriques 11, 12, 13, 14, 15, 16 sont reliées aux différentes électrodes comme détaillé ci-dessous. Dans l'exemple illustré sur les figures 2-3, une feuille 8, 9 en matériau électriquement isolant et flexible est placée respectivement sur chacune des faces de la feuille de matériau diélectrique 7 afin de protéger les électrodes 1, 2, 3, 4 et les pistes conductrices 11, 12, 13, 14, 15, 16. Les feuilles 8, 9 sont par exemple des feuilles de kapton d'épaisseur comprise entre 50 et 100 microns. Le kapton a une constante diélectrique de 110 kV/mm.

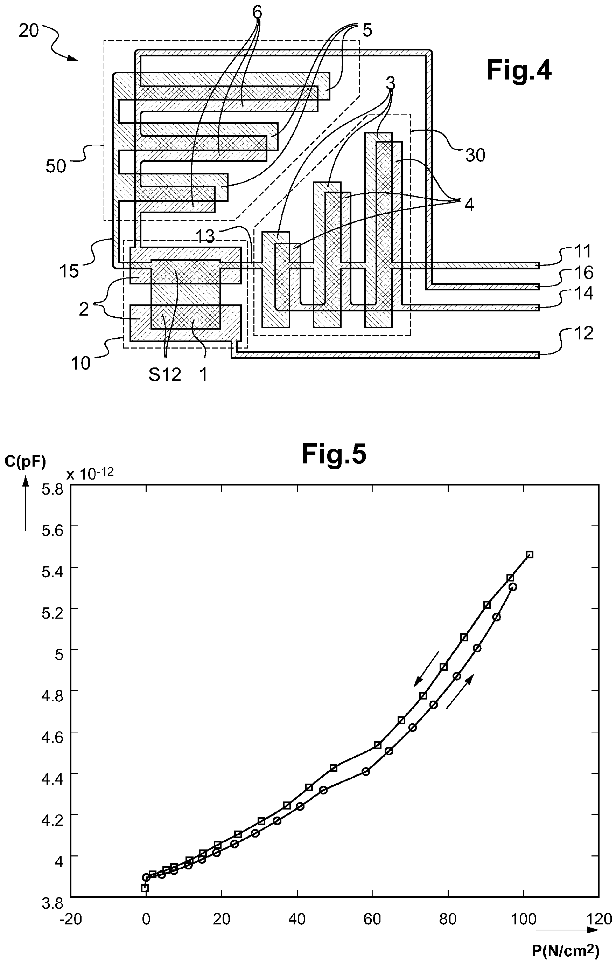

Le premier capteur capacitif 10 comporte une première électrode 1 fixée sur la première face de la feuille de matériau diélectrique 7 et une deuxième électrode 2 fixée sur la deuxième face de la feuille de matériau diélectrique 7. Dans l'exemple illustré sur la figure 1, la première électrode 1 et la deuxième électrode 2 sont de forme carrée. D'autres formes d'électrodes sont envisageables, comme décrit par exemple en lien avec la figure 4. Ici, la surface de la première électrode 1 est plus grande que la surface de la deuxième électrode 2. Sur les figures 1 et 2, en absence de forces de cisaillement suivant la direction X ou Y, l'étendue de la surface de la première électrode 1 recouvre entièrement l'étendue de la surface de la deuxième électrode 2. Par exemple, l'électrode 1 est un carré de 5 mm de côté et l'électrode 2 est un carré de 4 mm de côté, centré sur l'électrode 1. En absence de force de compression et de force de cisaillement, la première électrode 1 est séparée de la deuxième électrode 2 par la feuille de matériau diélectrique 7 d'épaisseur L. De préférence, l'épaisseur L est inférieure à 2 mm, par exemple l'épaisseur L est égale comprise entre 0.2 mm et 1,5 mm.

La première électrode 1 est reliée à une piste 11 électriquement conductrice déposée sur la première face du matériau diélectrique. De manière similaire, la deuxième électrode 2 est reliée à une autre piste 12 électriquement conductrice déposée sur la deuxième face du matériau diélectrique. La première électrode 1 et de la deuxième électrode 2, séparées par le matériau diélectrique 7, définissent un condensateur dont la surface S12 est déterminée par les surfaces en vis-à-vis de la première électrode 1 et de la deuxième électrode 2. Dans ce mode de réalisation, au repos, la surface S12 est égale à la plus petite des surfaces des deux électrodes 1, 2 carrées. Sur les figures 2 et 3, les traits en tirets verticaux entre les électrodes 1 et 2 délimitent l'étendue du condensateur formé entre ces électrodes. Les pistes conductrices 11 et 12 sont décalées dans le plan XY afin de ne pas contribuer à la valeur de la mesure du condensateur formé entre les électrodes 1 et 2.

En absence de force de pression ou de cisaillement, la valeur de la capacité du premier capteur capacitif 10 est déterminée par l'épaisseur L du diélectrique 7 et par la surface S12, par application de l'équation (I). Lorsqu'une force de compression est appliquée sur le capteur de pression 10 le long de la direction Z, l'épaisseur du diélectrique 7 varie de $\Delta L$ tandis que la surface S12 reste constante. La capacité du capteur capacitif 10 varie ainsi en fonction de la variation d'épaisseur $\Delta L$ du diélectrique 7. Le capteur capacitif 10 permet ainsi de mesurer la force de compression suivant la direction Z. Sur la figure 3, on a représenté une vue en coupe du capteur de pression suivant la ligne de coupe AA suite à l'application d'une force de cisaillement F appliquée dans la direction X sur la première face du diélectrique 7. La force de cisaillement F induit un déplacement latéral $\Delta X$ relatif entre la première face du diélectrique 7 et la deuxième face du diélectrique 7 suivant la direction X. Simultanément, la force de cisaillement F induit une compression $\Delta L$ de l'épaisseur du diélectrique dans la direction Z. Toutefois, la première électrode 1 étant plus étendue que la deuxième électrode 2, la surface S12 en regard des électrodes 1 et 2 reste constante. Par conséquent, le premier capteur capacitif 10 est sensible uniquement à la variation $\Delta L$ d'épaisseur du diélectrique. Une calibration de la valeur de la capacité du premier capteur capacitif 10 permet ainsi d'en déduire une mesure de la variation $\Delta L$ d'épaisseur du diélectrique. Cette mesure est prise entre les pistes conductrices 11 et 12.

Le deuxième capteur capacitif 30 comporte une première électrode 3 fixée sur la première face de la feuille de matériau diélectrique 7 et une deuxième électrode 4 fixée sur la deuxième face de la feuille de matériau diélectrique 7. Dans l'exemple illustré sur la figure 1, la première électrode 3 et la deuxième électrode 4 sont en forme de peignes, les dents du peigne étant disposées à équidistance le long de la direction X et s'étendant le long de la direction Y. Par exemple, la première électrode 3 et la deuxième électrode 4 comprennent chacune trois dents de longueur croissante suivant la direction Y. Sur la figure 1, au repos, les dents de la première électrode 3 sont décalées par rapport aux dents de la deuxième électrode 4 suivant la direction X et se recouvrent partiellement. La première électrode 3 est reliée à une piste 13 électriquement conductrice déposée sur la première face du matériau diélectrique. La piste 13 est reliée à la première électrode 1 du premier capteur de pression 10. La deuxième électrode 4 est reliée à une autre piste 14 électriquement conductrice déposée sur la deuxième face du matériau diélectrique. La première électrode 3 et de la deuxième électrode 4, séparées par le matériau diélectrique 7, définissent un condensateur dont la surface S34 est déterminée par les surfaces en vis-à-vis de la première électrode 3 et de la deuxième électrode 4. La surface S34, définie par l'intersection de la projection de la première électrode 3 sur la deuxième électrode 4, détermine la surface du condensateur formé par les électrodes 3 et 4 séparées par le diélectrique 7. Sur les figures 2 et 3, les traits en tirets verticaux entre les électrodes 3 et 4 délimitent l'étendue du condensateur formé entre ces électrodes. Les pistes conductrices 13 et 14 sont décalées dans le plan XY afin de ne pas contribuer à la mesure de variation de la capacité du condensateur formé entre les électrodes 3 et 4. Dans la direction Y, l'électrode 3 s'étend au-delà des extrémités de l'électrode 4. La forme et la disposition des électrodes 3 et 4 en peigne aux dents de longueur croissante permettent de maximiser la surface S34 pour un encombrement minimum de la cellule de capteurs 20. L'augmentation du nombre de dents permet d'augmenter la précision des mesures en augmentant la valeur de la variation de surface $\Delta S34$ pour une même valeur de force de cisaillement et de diminuer relativement l'effet des capacités parasites.

Les capacités parasites peuvent avoir plusieurs origines. Premièrement, le capteur lui-même comporte des capacités parasites formées entre des pistes conductrices parallèles. En effet, deux pistes conductrices de cuivre, imprimées côte à côte, forment une capacité où la surface mise en regard est égale à la longueur de la piste conductrice multipliée par l'épaisseur de la couche de cuivre. Deuxièmement, des capacités parasites sont introduites par le circuit électronique. Enfin, un autre parasite est introduit lors d'un contact extérieur, par exemple avec un doigt de l'utilisateur, à la surface du kapton. Pour remédier à cela, un blindage, constitué d'une plaque de cuivre ou d'aluminium, est disposé sur chaque face extérieure au capteur.

En absence de force de pression ou de cisaillement, la valeur de la capacité du deuxième capteur capacitif 30 est déterminée par l'épaisseur L du diélectrique 7 et par la surface S34, par application de l'équation (I). Lorsqu'une force de compression est appliquée sur le capteur de pression 30 le long de la direction Z, l'épaisseur du diélectrique 7 varie de ΔL tandis que la surface S34 reste constante. La capacité du capteur capacitif 30 varie ainsi en fonction de la variation d'épaisseur ΔL du diélectrique 7.

Comme illustré sur la figure 3, lorsqu'une force de cisaillement est appliquée dans la direction X sur la première face du diélectrique 7, le capteur de pression 30 subit un décalage latéral relatif ΔX entre la première électrode 3 et de la deuxième électrode 4 dans la direction X. Le décalage latéral ΔX induit une variation de la surface S34, qui diminue lorsque la force de cisaillement est appliquée dans le sens des X positifs et qui augmente lorsque la force de cisaillement est appliquée dans le sens des X négatifs. Toutefois, même en absence de composante de force de compression, une force de cisaillement induit, simultanément avec le décalage latéral ΔX, une déformation en épaisseur ΔL du matériau diélectrique 7 élastiquement déformable.

La mesure de la variation de capacité électrique du deuxième capteur capacitif de pression 30 est donc sensible à la fois à une force de compression le long de l'axe Z et à une force de cisaillement appliquée le long de l'axe X. Néanmoins, la combinaison de la mesure du premier capteur 10 et de la mesure du capteur 30 permet de discriminer une force de pression normale d'une force de cisaillement appliquée le long de l'axe X.

Par contre, lorsqu'une force de cisaillement est appliquée le long de l'axe Y, la surface S34 du capteur capacitif de pression 30 reste invariable, du fait que l'électrode 3 s'étend sur une plus grande longueur que l'électrode 4.

Ainsi, le deuxième capteur capacitif de pression 30 est sensible à un décalage latéral relatif suivant l'axe X entre les électrodes 3 et 4 et insensible à un décalage latéral relatif suivant l'axe Y entre les électrodes 3 et 4. Le capteur capacitif de pression 30 est aussi sensible à une variation d'épaisseur ΔL suivant l'axe Z entre les électrodes 3 et 4.

La mesure des variations de valeur de la capacité du deuxième capteur capacitif 30 est prise entre la piste conductrice 11 et la piste conductrice 14. En effet, la piste conductrice 11 est reliée à la première électrode 1 du premier capteur 10, elle-même reliée via la piste conductrice 13 à l'électrode 3 du deuxième capteur 30.

De manière analogue et complémentaire, le troisième capteur capacitif 50 comporte une première électrode 5 fixée sur la première face de la feuille de matériau diélectrique 7 et une deuxième électrode 6 fixée sur la deuxième face de la feuille de matériau diélectrique 7. Dans l'exemple illustré sur la figure 1, la première électrode 5 et la deuxième électrode 6 sont en forme de peignes, les dents du peigne étant disposées à équidistance le long de la direction Y et s'étendant le long de la direction X. A titre d'exemple illustratif et nullement limitatif, la première électrode 5 et la deuxième électrode 6 comprennent chacune trois dents de longueur croissante suivant la direction Y. Sur la figure 1, au repos, les dents de la première électrode 5 sont décalées par rapport aux dents de la deuxième électrode 6 suivant la direction Y de manière à se recouvrir partiellement. La première électrode 5 est reliée à une piste 15 électriquement conductrice déposée sur la première face du matériau diélectrique. La piste 15 est reliée à la première électrode 1 du premier capteur de pression 10. La deuxième électrode 6 est reliée à une autre piste 16 électriquement conductrice déposée sur la deuxième face du matériau diélectrique. La première électrode 5 et de la deuxième électrode 6, séparées par le matériau diélectrique 7, définissent un condensateur dont la surface S56 est déterminée par les surfaces en vis-à-vis de la première électrode 5 et de la deuxième électrode 6. Plus précisément, la surface S56 est définie par l'intersection de la projection de la première électrode 5 sur la deuxième électrode 6. Cette surface S56 détermine la surface du condensateur formé par les électrodes 5 et 6 séparées par le diélectrique 7. Les pistes conductrices 15 et 16 sont décalées afin de ne pas contribuer à la mesure de variation de la capacité du condensateur formé entre les électrodes 5 et 6. Dans la direction X, l'électrode 5 s'étend au-delà des extrémités de l'électrode 6. La forme et la disposition des électrodes 5 et 6 en peigne aux dents de longueur croissante permettent de maximiser la surface S56 pour un encombrement minimum de la cellule de capteurs 20.

En absence de force de pression ou de cisaillement, la valeur de la capacité du troisième capteur capacitif 50 est déterminée par l'épaisseur L du diélectrique 7 et par la surface S56, par application de l'équation (I). Lorsqu'une force de compression est appliquée sur le capteur de pression 50 le long de la direction Z, l'épaisseur du diélectrique 7 varie de ΔL tandis que la surface S56 reste constante. La capacité du capteur capacitif 50 varie ainsi en fonction de la variation d'épaisseur ΔL du diélectrique 7.

Lorsqu'une force de cisaillement F est appliquée dans la direction Y sur la première face du diélectrique 7, le capteur de pression 50 subit une déformation en épaisseur ΔL et un décalage latéral relatif ΔY entre la première électrode 5 et de la deuxième électrode 6 dans la direction Y. Le décalage latéral ΔY induit une variation de la surface S56, qui diminue lorsque la force de cisaillement est appliquée dans le sens des Y positifs et qui augmente lorsque la force de cisaillement est appliquée dans le sens des Y négatifs. Le déplacement ΔY suivant l'axe Y dépend directement de l'épaisseur L du diélectrique, du coefficient de Poisson V, du module d'Young E, de la force F appliquée suivant Y et de l'aire A sur laquelle cette force est appliquée selon la formule suivante :

$$\Delta Y = 2(1+V)F*L/(E*A) \qquad (II)$$

Par exemple, avec une feuille de silicone, on mesure une variation de surface ΔS de l'ordre de 10% pour une force de cisaillement F de l'ordre de 50 Newtons appliquée suivant la direction Y sur une surface de 1cm².

La mesure de la variation de capacité électrique du troisième capteur capacitif de pression 50 est donc sensible à la fois à une force de compression le long de l'axe Z et à une force de cisaillement appliquée le long de l'axe Y. Néanmoins, la combinaison de la mesure du premier capteur 10 et de la mesure du troisième capteur 50 d'une même cellule permet de discriminer une force de pression normale d'une force de cisaillement appliquée le long de l'axe Y.

Par contre, lorsqu'une force de cisaillement est appliquée le long de l'axe X, la surface S56 du capteur capacitif de pression 50 reste invariable, du fait que l'électrode 5 s'étend sur une plus grande longueur que l'électrode 6.

Ainsi, le troisième capteur capacitif de pression 50 est sensible à un décalage latéral relatif suivant l'axe Y entre les électrodes 5 et 6 et insensible à un décalage latéral relatif suivant l'axe X entre les électrodes 5 et 6. Le capteur capacitif de pression 50 est aussi sensible à une variation d'épaisseur ΔL suivant l'axe Z entre les électrodes 5 et 6.

La mesure des variations de valeur de la capacité du troisième capteur capacitif de pression 50 est prise entre la piste conductrice 11 et la piste conductrice 16. En effet, la piste conductrice 11 est reliée à la première électrode 1 du premier capteur 10, elle-même reliée via la piste conductrice 15 à l'électrode 5 du troisième capteur 50.

Les trois capteurs 10, 30 et 50 étant disposés à proximité l'un de l'autre, on fait l'approximation que la variation d'épaisseur ΔL est identique pour les trois capteurs 10, 30, 50 d'une même cellule 20. La mesure des trois capacités électriques, respectivement du premier capteur capacitif de pression 10, du deuxième capteur capacitif de pression 30 et du troisième capteur capacitif de pression 50 permet d'en déduire l'intensité et la direction d'une force de compression appliquée le long de l'axe Z, d'une force de cisaillement appliquée le long de l'axe X et d'une force de cisaillement appliquée le long de l'axe Y. Une cellule 20 fournit ainsi 6 informations.

Par exemple, pour mesurer le cisaillement, on mesure la valeur qui est intéressante est C[X ou Y]/C[normal], la composante L étant considérée comme identique pour des capacités adjacentes, elle est éliminée. Notons C[X] la capacité du capteur 30, A[X] la surface effective du capteur 30, C[Y] la capacité du capteur 50, A[Y] la surface effective du capteur 50, et C[Normal] la capacité du capteur 10 et A[Normal] la surface effective du capteur 10. Par application de la formule (I) on a :

$$C[X]= \varepsilon^* A[X] / L[X]$$

$$C[Normal]= \varepsilon *A[Normal] / L[Normal]$$

Où ε représente la constante diélectrique du matériau diélectrique 7.

On considère L[X]=L[Normal] car les capteurs 10 et 30 sont très proches l'un de l'autre, et que la surface du capteur de pression normale 10, A[Normal], est constante.

On en déduit que :

$$C[X]/C[Normal]=A[X]/A$$

La mesure des variations du rapport entre la capacité du capteur de cisaillement 30 suivant l'axe X et la capacité du capteur de pression normale 10 entre un état initial et lors de l'application d'une force, permet d'en déduire la valeur de ΔX. Par application de la formule (II), on en déduit la mesure de la force de cisaillement F suivant l'axe X.

La cellule 20 de trois capteurs capacitifs est reliée à un système de mesure par seulement quatre pistes électriquement conductrices les pistes 11, 12, 13 et 14. La piste 11 est déposée sur la première face du diélectrique 7 tandis que les pistes 12, 14 et 16 sont déposées sur la deuxième face du diélectrique 7. Les électrodes opposées étant sur des faces distinctes, un design d'électrodes dans un seul plan peut être employé. Cette disposition des pistes conductrices simplifie le procédé de fabrication par comparaison aux designs d'électrodes multiniveaux, qui nécessitent plus d'étapes de fabrication. Dans les systèmes de l'art antérieur, on utilise généralement deux pistes électriques indépendantes pour chaque capteur capacitif, c'est-à-dire six pistes pour trois capteurs. L'arrangement de la cellule 20 permet de réduire le nombre de pistes électriques de et vers un système électronique de mesure.

La cellule est reliée à un système électronique de mesure via les pistes conductrices 11, 12, 13, 14. On mesure séquentiellement les capacités électriques du premier capteur 10, du deuxième capteur 30 et du troisième capteur 50. De façon particulièrement avantageuse, on mesure chaque piste avec une fréquence de 100 Hz.

Un inconvénient de la configuration de la cellule 20, est que le potentiel appliqué sur l'électrode 1 risque de ne pas être uniforme sur toute la surface des électrodes 1, 3 et 5 reliées en série, ce qui affecte potentiellement la précision des mesures.

Pour une force de pression normale de 100 Newtons, on mesure une variation de la capacité du capteur 10 de l'ordre de 50%, comparée à la mesure de la même capacité au repos.

Pour une force de cisaillement de 50 Newtons suivant l'axe X, on mesure une variation de la capacité du capteur 30 de l'ordre de 10%, par comparaison à la mesure de la même capacité au repos.

On choisit le matériau diélectrique en fonction des applications. La feuille de matériau diélectrique doit rester dans sa zone d'élasticité pour des pressions appliquées comprises entre 0 et 10 kg/cm$^2$ (correspondant aux moyennes de la pression plantaire) et l'écrasement maximal doit être inférieur à 50% lorsque la pression appliquée est de 10kg/cm$^2$. Ainsi lorsque l'on mesure la capacité en sortie des électrodes, respectivement avant et après l'application d'une force de pression, il est possible de calculer la variation d'épaisseur du matériau diélectrique, par exemple du silicone. Par application de la loi de Hook, dans la zone d'élasticité du matériau, on déduit de la variation d'épaisseur L une mesure de la pression appliquée. En effet la loi de Hook indique que la pression normale $\sigma$ appliquée en surface du matériau est égale au produit du module d'Young E du matériau par le pourcentage $\Delta L/L$ de déformation en épaisseur du matériau :

$$\sigma = E.\frac{\Delta L}{L} \qquad\qquad \text{(III)}$$

De préférence, on utilise un matériau diélectrique élastiquement déformable en compression et en cisaillement, et qui reste dans sa zone d'élasticité pour une pression normale comprise entre 0 et 10 kg/cm$^2$. A la pression maximum de 10 kg/cm$^2$, l'écrasement du matériau est de préférence limité entre 10% et 50% de l'épaisseur. On définit une gamme de de valeurs théoriques du module d'Young comprise entre 30 kg/cm$^2$ et 100 kg/cm$^2$. Concernant les caractéristiques déterminées empiriquement, le matériau diélectrique doit avoir une force de tension (tensile strength) définie par la pression appliquée maximale avant rupture, comprise entre 20 et 100 MPa.

Dans un autre exemple de réalisation, on choisit pour diélectrique 7 une feuille d'uréthane de 1 mm d'épaisseur, fourni par la société Grainger (https://www.grainger.com/product/Foam-Sheet-13C455?functionCode=P2IDP2PCP). La mousse d'uréthane convient tout particulièrement pour la mesure de la pression normale. L'uréthane a l'avantage d'avoir un bon écrasement donc de permettre une bonne résolution spatiale de mesure. De plus, l'uréthane présente un faible hystérésis.

Dans un autre exemple de réalisation, on choisit pour diélectrique 7 une feuille de mousse de polyuréthane. Ce matériau a l'avantage de présenter une hystérésis très réduit, autour de 6%. Ce matériau se prête en particulier bien aux applications de mesure essentiellement de la pression normale.

Sur la figure 5, on a représenté une mesure de variation de capacité C (en pF) en fonction d'une force de pression normale P (en N/cm$^2$) appliquée sur un capteur de pression selon un exemple de réalisation de l'invention, en appliquant une force de pression croissante puis décroissante. Le matériau diélectrique est ici de l'uréthane. Ces mesures illustrent bien l'effet d'hystérésis du capteur, qui reste néanmoins limité à moins de 7% pour l'uréthane. Cet effet d'hystérésis peut produire des incertitudes importantes sur les mesures, car on ne dispose en général pas de l'information pour déterminer si on se place sur la courbe de pression croissante ou décroissante.

Dans un autre exemple, le diélectrique 7 est en mousse de polyuréthane, de 1mm d'épaisseur. La mousse de polyuréthane se prête tout particulièrement bien aux applications à la mesure des forces de pression normale.

Dans un exemple de réalisation, on choisit pour diélectrique 7 un élastomère, par exemple une feuille de silicone de 1mm d'épaisseur, ayant une dureté comprise entre 10 et 20 de la marque Nusil. La dureté est liée au module d'Young E par une relation empirique. Dans un autre exemple de réalisation, on choisit pour diélectrique 7 une feuille de silicone d'épaisseur 0,5mm, par exemple de la société Nusil, référence MED 4901. Le silicone se prête tout particulièrement bien aux applications à la mesure des forces de cisaillement. L'inconvénient du silicone est qu'il présente une hystérésis important, qui se situe autour de 30%.

Dans un autre exemple de réalisation, on choisit pour diélectrique 7 une feuille de liège qui a l'avantage d'avoir un module de Poisson extrêmement faible (V=0).

Les électrodes et les pistes conductrices sont par exemple déposées ou imprimées sur deux feuilles 8, 9 ultraminces de kapton. Une feuille 9 de kapton portant les électrodes 1, 3, 5 et les pistes conductrices 11, 13, 15 est collée sur une face de la feuille de diélectrique 7. Une autre feuille 8 de kapton portant les électrodes 2, 4, 6 et les pistes conductrices 12, 14, 16 est collée sur l'autre face de la feuille de diélectrique 7.

Sur la figure 4, on a représenté une variante d'une cellule 20 de capteurs de pression. Les mêmes signes de référence désignent des éléments identiques à ceux de la figure 1. En particulier, la structure et le fonctionnement des capteurs 30 et 50 sont identiques à ceux des capteurs décrits en lien avec la figure 1. Dans la variante de la figure 4, la forme et les dimensions des électrodes 1, 2 du premier capteur de pression 10 sont différentes de celles du capteur 10 de la cellule illustrée sur la figure 1. Dans la variante de la figure 4, la première électrode 1 est formée d'un carré dont la longueur du côté est inférieure aux dimensions de l'électrode 2. L'électrode 2 est formée de deux portions d'électrodes reliées en série. Par exemple, l'électrode 2 est formée de deux rectangles séparés par une tranchée s'étendant le long de la direction X et reliés en série par une piste conductrice. La surface de la première électrode 1 ne recouvre pas totalement la surface de la deuxième électrode 2. Ici, la surface de la première électrode est inférieure à la surface de la deuxième électrode 2. La surface S12 du condensateur formé par le diélectrique entre les électrodes 1 et 2, est ici constituée de deux rectangles et non pas d'un carré. D'autre part, la piste conductrice 16 n'est pas dans le prolongement d'un rectangle du peigne des électrodes 6. Néanmoins, le fonctionnement du capteur 10 de la figure 4 est analogue à celui du capteur 10 de la figure 1.

De manière générale, les électrodes 1 et 2 du premier capteur sont configurées de manière à ce que, lorsque le capteur 10 est soumis à une force de cisaillement le long de la direction X ou Y, dans la limite de la déformation élastique du matériau diélectrique 7, la surface S12 reste constante. De manière évidente pour l'homme du métier, la structure et la forme des électrodes 1 et 2 sont interchangeables. De même, les électrodes 3 et 4 sont interchangeables, ainsi que les électrodes 5 et 6.

Dans un exemple de réalisation, une cellule de capteurs 20 occupe une surface carrée de 1 cm de côté. Il est possible d'intégrer un grand nombre de cellules de capteurs 20 sur une même feuille de matériau diélectrique pour former un réseau de capteurs à haute résolution spatiale. Plus la surface d'une cellule est petite, plus la résolution spatiale (nombre de capteurs par cm$^2$) augmente. Néanmoins, on est limité par l'épaisseur du diélectrique 7 actuellement disponible sur le marché. En effet, si on veut diminuer la taille du capteur, il faut diminuer les surfaces S mises en regard, or l'épaisseur L doit être petite devant la grandeur caractéristique de S pour pouvoir se placer dans l'approximation d'un condensateur plan.

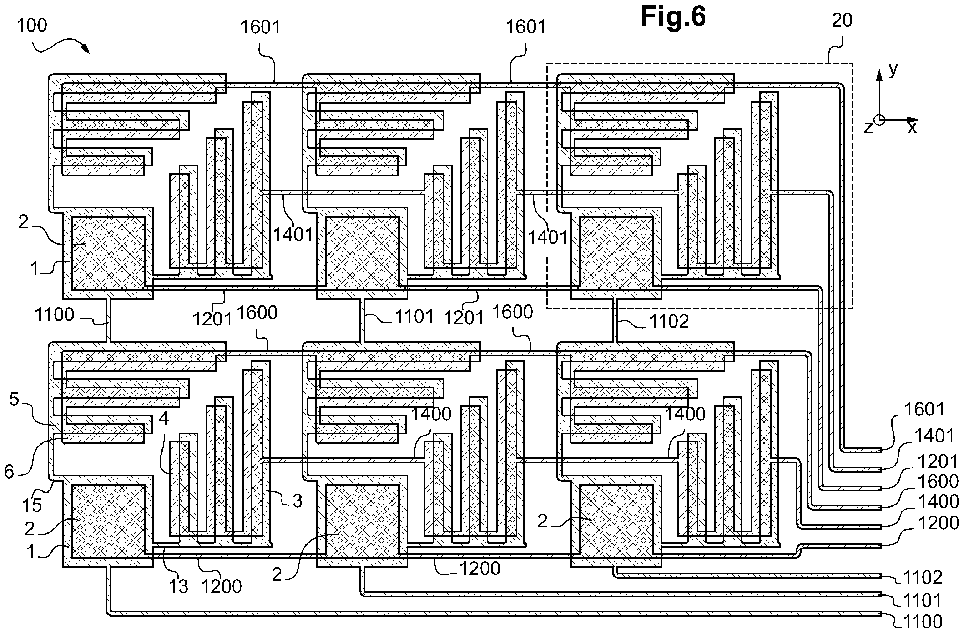

Sur la figure 6 on a représenté en vue de dessus un exemple de réseau de capteurs de pression. Ce réseau 100 comprend un ensemble de cellules 20 de capteurs de pression, telles que décrites en lien avec la figure 1, ces cellules étant disposées en matrice sur une même feuille mince de matériau diélectrique 7 élastiquement déformable. De façon particulièrement avantageuse, les cellules 20 sont disposées suivant des lignes et des colonnes.

Sur la figure 6, comme sur la figure 1, on a représenté en projection les électrodes et les pistes conductrices du réseau de capteurs de pression. Le réseau 100 comprend par exemple deux lignes et trois colonnes de cellules de plusieurs capteurs de pression. Par exemple, les électrodes sont fabriquées par une technique de circuit imprimé (PCB) et le réseau est développé en utilisant un logiciel Eagle. Dans ce cas, les cellules 20 d'un même réseau sont de préférence identiques les unes aux autres. La structure d'une cellule 20 est identique à celle décrite en lien avec la figure 1. De manière générale, une cellule 20 comporte des électrodes 1, 3 et 5 sur une face du matériau diélectrique 7 et des électrodes 2, 4 et 6 sur l'autre face du matériau diélectrique 7. Les électrodes 1 et 2 forment un capteur capacitif de pression normale, sensible aux variations en épaisseur ΔL du matériau diélectrique 7. Les électrodes 3 et 4, en forme de peigne, décalées l'une par rapport à l'autre, forment un capteur capacitif de pression en cisaillement sensible à une force de cisaillement appliquée le long de la direction X. Les électrodes 5 et 6, en forme de peigne, décalées l'une par rapport à l'autre, forment un capteur capacitif de pression en cisaillement sensible à une force de cisaillement appliquée le long de la direction Y. De façon avantageuse, l'électrode 1 est reliée à l'électrode 3 par une piste conductrice 13. De manière analogue, l'électrode 1 est reliée à l'électrode 5 par une piste conductrice 15. Ainsi, une cellule 20 de capteurs de pression commandée par seulement quatre pistes conductrices externes permet de fournir six informations de pression, c'est-à-dire des mesures de pression en 3D, avec indication du sens de la pression appliquée.

Le réseau 100 comporte ici plusieurs cellules à capteurs de pression disposées en ligne et en colonnes.

Une piste conductrice 1100 est reliée à l'électrode 1 d'une première cellule située à l'intersection d'une première ligne et d'une première colonne. Cette piste 1100 se prolonge pour relier électriquement l'électrode 5 d'une cellule à capteurs de pression à l'électrode 1 d'une autre cellule immédiatement adjacente dans la même colonne. Ainsi, les électrodes 1, 3, et 5 de toutes les cellules de la première colonne sont reliées en série.

De manière similaire, une autre piste conductrice 1101 est reliée à l'électrode 1 d'une cellule dans une deuxième colonne du réseau 100. Cette piste 1101 se prolonge pour relier électriquement l'électrode 5 d'une cellule à capteurs de pression à l'électrode 1 d'une autre cellule immédiatement adjacente de la deuxième colonne. Ainsi, les électrodes 1, 3, et 5 de toutes les cellules 20 de la deuxième colonne sont reliées en série.

De même, une autre piste conductrice 1102 est reliée à l'électrode 1 d'une cellule dans une troisième colonne du réseau 100. Cette piste 1102 se prolonge pour relier électriquement l'électrode 5 d'une cellule à capteurs de pression à l'électrode 1 d'une autre cellule immédiatement adjacente de la troisième colonne. Ainsi, les électrodes 1, 3, et 5 de toutes les cellules 20 de la troisième colonne sont reliées en série.

Par ailleurs, une autre piste conductrice 1200 est reliée à l'électrode 2 de la première cellule située à l'intersection de la première ligne et de la première colonne. Cette piste 1200 se prolonge pour relier électriquement l'électrode 2 d'une cellule à capteurs de pression à l'électrode 2 d'une autre cellule immédiatement adjacente sur la première ligne, et ainsi de suite. Ainsi, les électrodes 2 de toutes les cellules de la première ligne sont reliées en série à la piste 1200.

Une autre piste conductrice 1400 est reliée à l'électrode 4 de la première cellule située à l'intersection de la première ligne et de la première colonne. Cette piste 1400 se prolonge pour relier électriquement l'électrode 4 d'une cellule à capteurs de pression à l'électrode 4 d'une autre cellule immédiatement adjacente sur la première ligne, et ainsi de suite. Ainsi, les électrodes 4 de toutes les cellules de la première ligne sont reliées en série à la piste 1400.

De manière analogue, une autre piste conductrice 1600 est reliée à l'électrode 6 de la première cellule située à l'intersection de la première ligne et de la première colonne. Cette piste 1600 se prolonge pour relier électriquement l'électrode 6 d'une cellule à capteurs de pression à l'électrode 6 d'une autre cellule immédiatement adjacente sur la première ligne, et ainsi de suite. Ainsi, les électrodes 6 de toutes les cellules de la première ligne sont reliées en série à la piste 1600.

De même, sur la deuxième ligne, les électrodes 2 de toutes les cellules de la deuxième ligne sont reliées en série à la piste 1201, les électrodes 4 de toutes les cellules de la deuxième ligne sont reliées en série à la piste 1401 et les électrodes 6 de toutes les cellules de la deuxième ligne sont reliées en série à la piste 1601.

On obtient ainsi un réseau comportant six cellules arrangées sur deux lignes et trois colonnes, qui est relié électriquement à un système extérieur de polarisation et de mesure par l'intermédiaire des pistes conductrices 1100, 1101, 1102, 1200, 1400, 1600, 1201, 1401, 1601, soit au total 9 pistes conductrices. Or chaque cellule de capteurs fournit six mesures, soit au total pour six cellules 36 mesures.

On adresse séquentiellement chaque cellule du réseau 100 de capteurs en sélectionnant une ligne et une colonne. Le système électronique est adapté pour mesurer des capacités entre 1 et 20 pF. L'architecture du réseau permet une très grande densité de capteurs capacitifs de pression, avec un nombre très réduit de pistes conductrices de liaison avec un système extérieur de mesure de capacité. Un réseau de capteurs dont chaque cellule de 3 capteurs occupe une surface de 1 cm de côté a été testé avec succès. Pour des capteurs de pression normale uniquement, on peut augmenter la densité jusqu'à 3 ou 4 capteurs par cm$^2$.

L'arrangement des capteurs permet d'utiliser toute la surface disponible, et de fournir des mesures de pression normale et de force de cisaillement avec une bonne sensibilité, et une bonne précision.

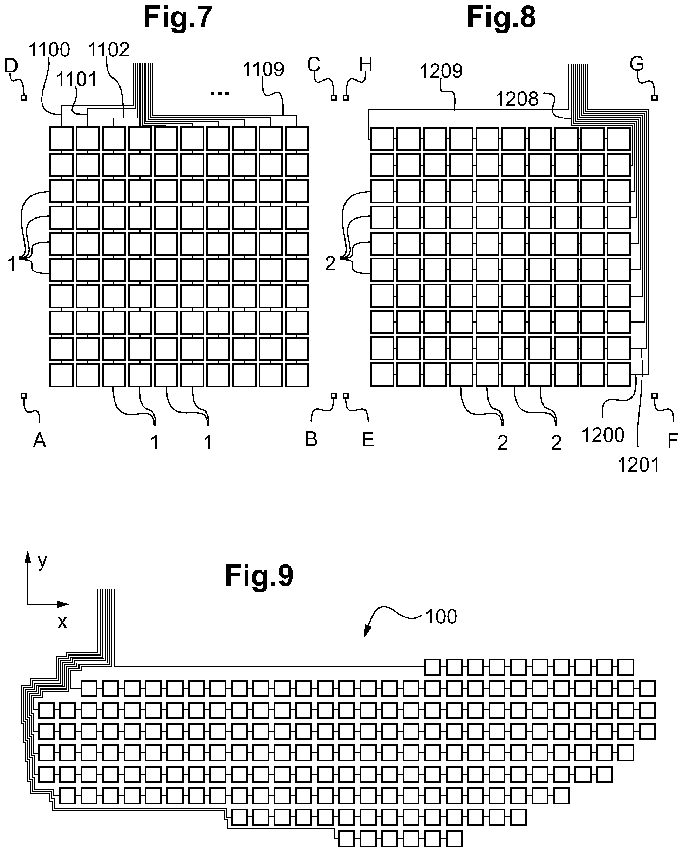

Sur la figure 7 on a représenté un exemple de schéma de réseau d'électrodes 1 destinées à être fixées sur une première face d'une feuille de matériau diélectrique 7. Les pistes conductrices 1100, 1101, 1102,..., 1109 relient en série les électrodes 1 par colonnes. Le réseau d'électrodes 1 et de pistes conductrices 1100, 1101, 1102,..., 1109 est imprimé sur un support 9, par exemple un circuit imprimé ou une feuille mince de kapton d'épaisseur comprise entre 50 et 100 microns. Une alternative au kapton est le polyester, qui a l'avantage d'être moins coûteux, mais de présenter une moindre résistance à la chaleur (80°C max). Des repères A, B, C, D sont imprimés simultanément sur le support 9, pour permettre l'alignement des électrodes par rapport à la feuille de matériau diélectrique 7, conformément au schéma électrique prévu.

Sur la figure 8 on a représenté un schéma de réseau d'électrodes 2 destinées à être fixées sur la face opposée du matériau diélectrique 7. Les pistes conductrices 1200, 1201, ..., 1208, 1209 relient en série les électrodes 2 par lignes. Le réseau d'électrodes 2 et de pistes conductrices 1200, 1201, ..., 1208, 1209 est imprimé sur un support 8, par exemple un circuit imprimé ou une autre feuille mince de kapton. Des repères E, F, G, H sont imprimés simultanément sur le support 8.

On place le support 9 sur une face du matériau diélectrique, puis on place le support 8 sur la face opposée du matériau diélectrique 7, de manière à aligner le repère C avec le repère H, respectivement le repère B avec le repère E, le repère D avec le repère G et enfin, le repère A avec le repère F.

Sur les figures 7 et 8, on observe que pour un réseau de 10 lignes et 10 colonnes, comportant donc 100 cellules de capteurs, il suffit de 20 pistes conductrices pour effectuer séquentiellement toutes les mesures de pression. Pour un réseau de 100 cellules à capteurs de pression en trois dimensions, correspondant à 300 capteurs, il suffit de relier le réseau par 40 pistes conductrices pour effectuer séquentiellement toutes les mesures de pression en compression et en cisaillement.

Les mesures de tous les capteurs d'un réseau de capteurs ne sont pas effectuées simultanément, contrairement aux systèmes où chaque capteur est adressé indépendamment.

Ce réseau de capteurs permet simultanément d'augmenter la densité de capteurs de pression, qui permet d'obtenir une bonne résolution spatiale, tout en limitant le nombre de pistes conductrices vers l'extérieur.

Sur la figure 9, on a représenté un schématiquement un réseau 100 de capteurs capacitifs de pression destiné à former une semelle. Le réseau comporte 188 capteurs disposés en 29 lignes et 9 colonnes dans le plan XY. Les capteurs sont disposés suivant un schéma prédéterminé correspondant à la forme générale d'un pied.

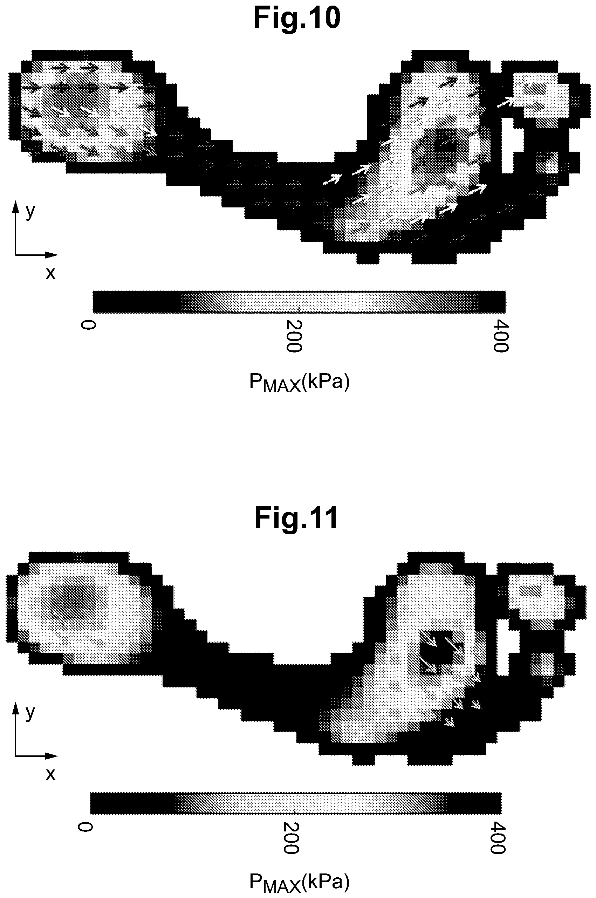

La figure 10 illustre schématiquement un exemple de représentation graphique des mesures de pression obtenues au moyen d'une semelle à capteurs tel représenté sur la figure 9. Sur la figure 10, la mesure de pression normale est représentée suivant des carrés, qui correspondent à la résolution spatiale de la semelle. Un code de couleur permet de représenter la répartition des valeurs de pression normale suivant la direction Z mesurées en fonction de la position en XY du capteur. La mesure de force de cisaillement est représentée simultanément sur la figure 10 sous forme de flèches : la direction des flèches représente la direction de la force de cisaillement, résultante de la mesure de la force de cisaillement suivant la direction X et suivant la direction Y. L'intensité de la mesure de la force de cisaillement est représentée ici en utilisant un code de couleurs pour les flèches.

La figure 11 illustre schématiquement un autre exemple de représentation graphique des mesures de pression obtenues au moyen d'une semelle à capteurs tel représentée sur la figure 9. Les mesures de pression normale sont représentées de manière analogue à la figure 10, par des carrés dont la couleur est codée en fonction de l'amplitude de la force de compression suivant la direction Z mesurées en fonction de la position en XY. La mesure de force de cisaillement est représentée simultanément sur la figure 11 sous forme de flèches : la direction des flèches représente la direction de la force de cisaillement, résultante de la mesure de la force de cisaillement suivant la direction X et suivant la direction Y. La longueur des flèches représente ici l'intensité de la mesure de la force de cisaillement.

La représentation graphique des mesures de pression fournit à l'utilisateur la répartition des forces de pression sur la surface du capteur avec une résolution de plusieurs dizaines de capteurs sur une semelle. Le capteur fournit pour chaque capteur élémentaire, l'intensité, la direction et le sens de la force appliquée au centre de chaque cellule de capteurs, d'où l'intérêt d'avoir un maximum de capteurs et une résolution spatiale la plus fine possible. Le réseau de capteur permet de déterminer précisément le point d'application de la force de pression.

La représentation des mesures de pression illustrée sur les figures 10 et 11 permet immédiatement à un utilisateur sans qualification particulière de connaître la répartition spatiale des forces de pression sur la semelle à réseau de capteurs.

Les mesures peuvent être répétées avec une cadence élevée, ce qui permet de corriger une posture inadaptée.

Dans le domaine médical, un réseau de capteurs intégré à une semelle trouve notamment des applications comme outil de diagnostic pour les professionnels du pied (podologues, podo-othésistes...), comme outil d'aide à la rééducation physique, ou comme semelle de prévention portée au quotidien par un patient diabétique atteint de neuropathie. Un réseau de capteurs de pression peut être intégré à un tapis comme outil de diagnostic du podologue. Intégré à un linge ou un vêtement sur une surface plus importante, le système à réseau de capteurs peut être utilisé comme nappe de prévention des escarres pour les personnes handicapées. Placé sur les mains courantes des roues des fauteuils roulants, un système à réseau de capteurs permet de quantifier l'interaction avec les mains des hémiplégiques. La détection des points d'appui du corps ou du pied d'un patient et la mesure de la répartition des forces de pression peuvent permettre d'améliorer la prévention des escarres ou la prévention des blessures du pied pour les personnes souffrant de diabète.

Une semelle à réseau de capteurs peut aussi trouver des applications comme outil de mesure pour les vendeurs de chaussures personnalisées (sportifs, chaussures de luxe...).

De façon particulièrement avantageuse, le système à réseau de capteurs est connecté par liaison sans fil à un boîtier de contrôle.

Dans le domaine sportif, une semelle à réseau de capteurs portée par un sportif, lui permet de quantifier sa course.

Les applications de l'invention ne se limitent nullement aux applications dans le domaine médical ou sportif, les capteurs de mesure de pression peuvent aujourd'hui être connectés à un téléphone intelligent et trouver des applications d'automesure destinées au grand public.

Dans le domaine récréatif, une semelle à réseau de capteurs de pression peut être connectée à un jeu vidéo pour remplacer une manette de jeu.

D'autres applications récréatives sont envisagées, telles qu'un tapis de danse connecté, ou un détecteur de choc sur les casques de football américain.

Procédé

Différents procédés de fabrication sont envisagés pour fabriquer un réseau de capteurs de pression dans le cadre de l'invention.

Dans un premier mode de réalisation, on utilise la technologie classique des circuits imprimés (PCB) de préférence sur support flexible (FPCB). Suivant cette technique, les électrodes sont fabriquées sur des supports 8 et 9 électriquement isolants, qui sont ensuite fixés sur les faces opposées d'une feuille de matériau diélectrique 7. De préférence, les électrodes et les pistes conductrices sont en cuivre ou en argent.

Comme indiqué plus haut, pour chaque support, la technique de fabrication repose sur un seul niveau d'électrodes. La fabrication des pistes conductrices sur un seul niveau est rapide et produit un circuit électrique plus fiable qu'un circuit électrique à plusieurs niveaux reliés par des vias. Cet avantage est décisif dans les applications visées où le réseau de capteurs est soumis à des forces de compression et/ou de cisaillement relativement importantes, par comparaison aux applications tactiles.

On obtient ainsi un système comportant une feuille mince de diélectrique 7 prise en sandwich entre deux supports flexibles 8 et 9, les électrodes étant fixées sur les faces opposées du diélectrique et protégées par les supports flexibles 8, 9.

Une liaison filaire ou sans fil permet de relier électriquement les pistes conductrices du réseau de capteurs à un système externe de mesure.

**EP 3 169 983 B1**

Dans un autre mode de réalisation, on imprime directement les électrodes et les pistes conductrices sur une feuille mince de matériau diélectrique. A cet effet, on utilise une avantageusement une imprimante à encre métallique (par exemple de type Fujifilm Dimatix Materials Printer DMP-2800 Series) pour imprimer les électrodes 1, 3, 5 et les pistes conductrices 11, 13, 15, 1100, 1101, ...1109 sur une première face du matériau diélectrique, puis on imprime imprimer les autres électrodes 2, 4, 6 et les autres pistes conductrices 12, 14, 16, 1200, 1201, ...1209, 1400, ..., 1600, 1601, ..., sur l'autre face du matériau diélectrique. De préférence, on utilise une encre métallique composée de cuivre ou d'argent. A titre d'exemple, l'encre est une encre à l'argent de la société Inktec, référence TEK-IJ-020, composée de 20% d'argent en poids. Ce procédé est plus rapide que le procédé passant par un PCB. Actuellement moins précis qu'un procédé PCB mais encore assez coûteux, il est fort probable que le coût de la technique d'impression à encre métallique baisse et que la précision de l'impression à encre métallique s'améliore nettement dans les prochaines années.

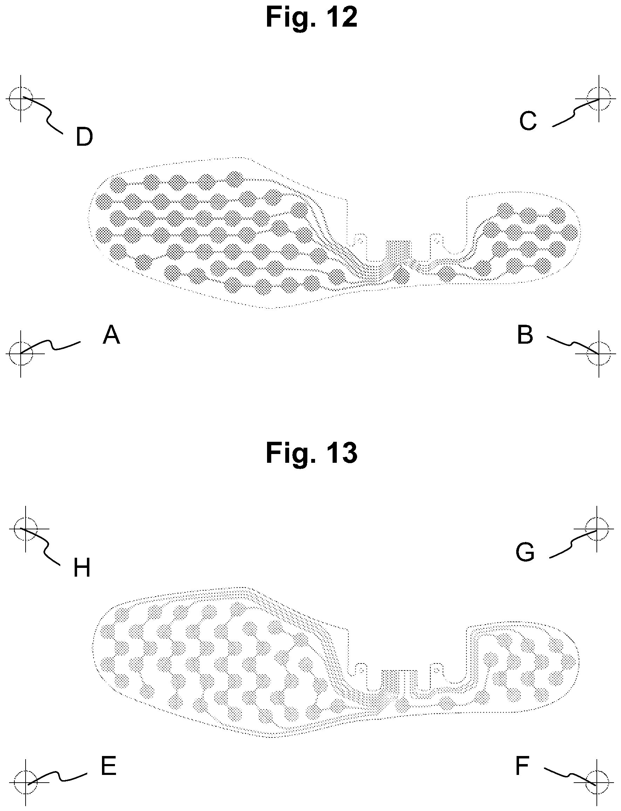

Dans un autre mode de réalisation, illustré sur les figures 12 et 13, on fabrique une semelle comprenant uniquement des capteurs de pression. On imprime les électrodes et les pistes conductrices par sérigraphie monocouche sur une feuille mince de PET ayant une épaisseur d'environ 100 microns. On imprime les premières électrodes telles que représentées sur la figure 12 et les deuxièmes électrodes telles que représentées sur la figure 13.

Dans un exemple de réalisation, les premières électrodes et les deuxièmes électrodes sont imprimées dans deux zones adjacentes sur une même feuille de PET. Dans ce cas, les premières électrodes et les deuxièmes électrodes peuvent être imprimées simultanément. Au voisinage de chaque motif d'électrode, on imprime aussi sur la feuille de PET des repères en forme de croix permettant l'alignement des motifs d'électrodes.

On découpe la feuille de PET pour séparer, d'une part, le motif des premières électrodes et, d'autre part, le motif des deuxièmes électrodes. Dans un autre exemple de réalisation, les premières électrodes et les deuxièmes électrodes sont imprimées respectivement sur deux feuilles distinctes de PET.

On colle une feuille de PET portant le motif des premières électrodes sur une face d'une feuille de matériau diélectrique élastiquement déformable, par exemple de mousse élastomère de polyuréthane ayant une épaisseur d'environ 1 mm. On colle une autre feuille de PET portant le motif des deuxièmes électrodes sur l'autre face de la feuille de mousse élastomère de polyuréthane. De façon avantageuse, la feuille de mousse élastomère de polyuréthane comporte des ouvertures permettant d'aligner les repères en forme de croix disposés sur la feuille portant les premières électrodes par rapport aux repères en forme de croix de l'autre feuille portant les deuxièmes électrodes. Ainsi, on obtient un alignement initial des première et deuxième électrode de chaque capteur de pression. Après séchage de la colle, on découpe la forme extérieure de la semelle.

Dans l'exemple illustré sur les figures 12-13, la semelle comporte 60 capteurs de pression. Les premières électrodes sont reliées en série par 11 lignes formant 11 pistes conductrices destinées à être reliées à un système électronique. Les deuxièmes électrodes sont reliées en série par 12 colonnes formant 12 pistes conductrices destinées à être reliées à un système électronique. Chaque capteur de pression représenté par un disque est à l'intersection d'une ligne et d'une colonne, permettant ainsi l'adressage de chacun des 60 capteurs de pression individuellement. Le système est relié uniquement par un total de 11+ 12 = 23 lignes conductrices. Cette semelle offre ainsi un réseau de capteurs de pression ayant une densité et une résolution spatiale élevée, tout en limitant le nombre de liaisons électriques.

Dans un autre exemple de réalisation, on fabrique une semelle de capteurs comprenant 69 capteurs de pression, disposés suivant 11 lignes et 16 colonnes, comportant donc au total seulement 27 liaisons électriques.

Bien entendu, la disposition des capteurs de pression, des lignes et des colonnes peut être variée à volonté, pour être adaptée à la taille du pied et aux besoins spécifiques.

**Revendications**

**1.** Système à réseau de capteurs de pression et de cisaillement comprenant :

- une feuille de matériau diélectrique (7) élastiquement déformable en compression et en cisaillement, la feuille de matériau diélectrique (7) ayant une première face et une deuxième face,

**caractérisé en ce que** le système à réseau de capteurs comporte :

- un réseau (100) comprenant une pluralité de cellules de capteurs de pression ( 20), les cellules (20) étant disposées suivant au moins trois lignes et au moins trois colonnes, chaque cellule (20) comprenant un premier capteur capacitif de pression normale (10) suivant une première direction (Z), et dans lequel au moins une cellule (20) du réseau (100) comporte en outre un deuxième capteur capacitif de cisaillement (30) suivant une deuxième direction (X) et un troisième capteur capacitif de cisaillement (50) suivant une troisième direction (Y), - chaque capteur capacitif (10, 30, 50) étant constitué d'une première électrode (1, 3, 5) fixée sur la première face de la feuille de matériau diélectrique (7) et d'une deuxième électrode (2, 4, 6) fixée sur la deuxième face

13

de la feuille de matériau diélectrique (7),
- lesdites premières électrodes (1, 3, 5) des capteurs capacitifs (10, 30, 50) d'une cellule (20) étant reliée en série à une première piste électriquement conductrice (11) reliant une ligne de cellules du réseau de capteurs ;
- la deuxième électrode (2) du capteur capacitif de pression normale (10) d'une cellule (20) étant reliée à une deuxième piste (12) électriquement conductrice reliant une colonne de capteurs capacitifs de pression normale (10) du réseau de capteurs, la deuxième électrode (4) du capteur capacitif de cisaillement (30) suivant la deuxième direction (X) étant reliée à une troisième piste (14) électriquement conductrice reliant une ligne de capteurs capacitifs de cisaillement (30) suivant la deuxième direction (X) du réseau de capteurs et la deuxième électrode (6) du capteur capacitif de cisaillement (50) suivant la troisième direction (Y) étant reliée à une quatrième piste (16) électriquement conductrice reliant une colonne de capteurs capacitifs de cisaillement (50) suivant la troisième direction (Y) du réseau de capteurs, lesdites première et deuxième électrodes (3, 4, 5, 6) des capteurs capacitifs de cisaillement (30, 50) étant en forme de peigne,

et **en ce que** le système à réseau de capteurs comprend des moyens d'adressage adaptés pour mesurer la capacité électrique d'un capteur capacitif (10, 30, 50) situé à l'intersection d'une ligne et d'une colonne, ladite ligne correspondant à une première piste (11) reliée à ladite première électrode (1, 3, 5) et ladite colonne correspondant à une autre piste (12, 14, 16) reliée à l'une desdites deuxièmes électrodes (2, 4, 6).

2. Système à réseau de capteurs de pression et de cisaillement selon la revendication 1, dans lequel la feuille de matériau diélectrique (7) élastiquement déformable en compression et en cisaillement est en un matériau choisi parmi : un liège micro-architecturé, un élastomère, un caoutchouc, un uréthane, un silicone, un butyl rubber, un polymère, un néoprène, un polyuréthane, un polyisoprène ou une mousse d'uréthane.

3. Système à réseau de capteurs de pression et de cisaillement selon l'une des revendications 1 à 2, dans lequel ladite première électrode (1, 3, 5) et ladite première piste (11) électriquement conductrice sont imprimées sur une feuille (9) en matériau électriquement isolant et flexible, et, respectivement, dans lequel ladite deuxième électrode (2, 4, 6) et lesdites autres pistes (12, 14, 16) électriquement conductrices sont imprimées sur une autre feuille (8) en matériau électriquement isolant et flexible.

4. Système à réseau de capteurs de pression et de cisaillement selon l'une des revendications 1 à 3, dans lequel la première piste (11) électriquement conductrice et la deuxième piste (12) électriquement conductrice d'une cellule (20) sont reliées à un système électronique adapté pour mesurer une variation de la capacité électrique du capteur capacitif de pression normale (10), le système électronique étant adapté pour en déduire une force de pression normale appliquée sur ledit capteur capacitif de pression normale (10) le long de la première direction (Z).

5. Système à réseau de capteurs de pression et de cisaillement selon l'une des revendications 1 à 4, dans lequel la première piste (11) électriquement conductrice et la troisième piste (14) électriquement conductrice d'une cellule (20) sont reliées audit système électronique, qui est adapté pour mesurer une variation de la capacité électrique du deuxième capteur capacitif de force de cisaillement (30) suivant la deuxième direction (X), le système électronique étant adapté pour en déduire l'amplitude et le sens d'une force de cisaillement appliquée sur ledit capteur capacitif de force de cisaillement (20) le long de la deuxième direction (X).

6. Système à réseau de capteurs de pression et de cisaillement selon la revendication 5, dans lequel la première piste (11) électriquement conductrice et la quatrième piste (16) électriquement conductrice d'une cellule (20) sont reliées audit système électronique, qui est adapté pour mesurer une variation de la capacité électrique du troisième capteur capacitif de cisaillement (50) suivant la troisième direction (Y), le système électronique étant adapté pour en déduire l'amplitude et le sens d'une force de cisaillement appliquée sur ledit troisième capteur capacitif de cisaillement (50) le long de la troisième direction (Y).

7. Système à réseau de capteurs de pression et de cisaillement selon l'une des revendications 1 à 6, dans lequel lesdites pistes électriquement conductrices (11, 12, 14, 16) sont reliées à des moyens de mesure d'une variation de la capacité électrique de capteur capacitif (10, 30, 50) par des liaisons filaires ou sans fil.

8. Système à réseau de capteurs de pression et de cisaillement selon l'une des revendications 1 à 7 comprenant un dispositif d'affichage des mesures de force de pression et/ou de cisaillement dans lequel le dispositif d'affichage est configuré pour représenter graphiquement, en fonction de la disposition du réseau de capteur, la pression normale mesurée par chaque cellule du réseau de capteur et/ou simultanément l'amplitude et la direction de la force de cisaillement mesurée par chaque cellule du réseau de capteur.

**9.** Semelle pour chaussure comprenant un système à réseau de capteurs de pression et de cisaillement selon l'une des revendications 1 à 8.

**Patentansprüche**

**1.** System aus einem Netzwerk aus Druck- und Scherbelastungssensoren mit

- einem durch Druck und Scherung elastisch verformbaren Blatt (7) aus dielektrischem Material, wobei das Blatt (7) aus dielektrischem Material eine erste und eine zweite Seite aufweist,

**dadurch gekennzeichnet, daß** das System aus einem Netzwerk aus Sensoren

- ein Netzwerk (100) aufweist, das eine Anzahl Zellen aus Drucksensoren (20) aufweist, wobei die Zellen (20) in wenigstens drei Zeilen und in wenigstens drei Spalten angeordnet sind, wobei jede Zelle (20) einen ersten kapazitiven Sensor (10) für senkrechten Druck in einer ersten Richtung (Z) aufweist, und bei dem wenigstens eine Zelle (20) des Netzwerks (100) außerdem einen zweiten kapazitiven Sensor (30) für Scherung in einer zweiten Richtung (X) und einen dritten kapazitiven Sensor (50) für Scherung in einer dritten Richtung (Y) aufweist,
- wobei jeder kapazitive Sensor (10, 30, 50) aus einer auf der ersten Seite des Blatts (7) aus dielektrischem Material befestigten ersten Elektrode (1, 3, 5) und einer auf der zweiten Seite des Blatts (7) aus dielektrischem Material befestigten zweiten Elektrode (2, 4, 6) besteht,
- wobei die ersten Elektroden (1, 3, 5) der kapazitiven Sensoren (10, 30, 50) einer Zelle (20) in Reihe mit einer eine Zeile Zellen des Netzwerks aus Sensoren verbindenden ersten elektrisch leitenden Piste (11) verbunden sind,
- wobei die zweite Elektrode (2) des kapazitiven Sensors (10) für senkrechten Druck einer Zelle (20) mit einer eine Spalte kapazitiver Sensoren (10) für senkrechten Druck des Netzwerks aus Sensoren verbindenden zweiten elektrisch leitenden Piste (12) verbunden ist, wobei die zweite Elektrode (4) des kapazitiven Sensors (30) für Scherung in der zweiten Richtung (X) mit einer eine Zeile kapazitiver Sensoren (30) für Scherung in der zweiten Richtung (X) des Netzwerks aus Sensoren verbindenden dritten elektrisch leitenden Piste (14) verbunden ist und die zweite Elektrode (6) des kapazitiven Sensors (50) für Scherung in der dritten Richtung (Y) mit einer eine Spalte kapazitiver Sensoren (50) für Scherung in der dritten Richtung (Y) des Netzwerks aus Sensoren verbindenden vierten elektrisch leitenden Piste (16) verbunden ist, wobei die ersten und zweiten Elektroden (3, 4, 5, 6) der kapazitiven Sensoren (30, 50) in Form eines Kamms vorliegen,

und daß das System aus einem Netzwerk aus Sensoren Adressiermittel aufweist, die dazu ausgelegt sind, die elektrische Kapazität eines an einem Kreuzungspunkt einer Zeile und einer Spalte gelegenen kapazitiven Sensors (10, 30, 50) zu messen, wobei die Zeile einer mit der ersten Elektrode (1, 3, 5) verbundenen ersten Piste (11) entspricht und die Spalte einer mit einer der zweiten Elektroden (2, 4, 6) verbundenen anderen Piste (12, 14) entspricht.

**2.** System aus einem Netzwerk aus Druck- und Scherbelastungssensoren gemäß Anspruch 1, bei dem das durch Druck und Scherung elastisch verformbare Blatt (7) aus dielektrischem Material aus einem Material besteht, das aus einem Kork mit Mikrostruktur, einem Elastomer, einem Kautschuk, einem Urethan, einem Silikon, einem Butylgummi, einem Polymer, einem Neopren, einem Polyurethan, einem Polyisopren oder einem Urethanschaum ausgewählt ist.

**3.** System aus einem Netzwerk aus Druck- und Scherbelastungssensoren gemäß einem der Ansprüche 1 bis 2, bei dem die erste Elektrode (1, 3, 5) und die erste elektrisch leitende Piste (11) auf einem Blatt (9) aus elektrisch isolierendem und flexiblem Material aufgedruckt sind und bei dem jeweils die zweite Elektrode (2, 4, 6) und die anderen elektrisch leitenden Pisten (12, 14, 16) auf einem anderen Blatt (8) aus elektrisch isolierendem und flexiblem Material aufgedruckt sind.

**4.** System aus einem Netzwerk aus Druck- und Scherbelastungssensoren gemäß einem der Ansprüche 1 bis 3, bei dem die erste elektrisch leitende Piste (11) und die zweite elektrisch leitende Piste (12) einer Zelle (20) mit einem elektronischen System verbunden sind, das dazu ausgelegt ist, eine Änderung der elektrischen Kapazität des kapazitiven Sensors (10) für senkrechten Druck zu messen, wobei das elektronische System dazu ausgelegt ist, daraus eine auf den Sensor (10) für senkrechten Druck entlang der ersten Richtung (Z) ausgeübte Kraft senkrechten Drucks abzuleiten.

**5.** System aus einem Netzwerk aus Druck- und Scherbelastungssensoren gemäß einem der Ansprüche 1 bis 4, bei dem die erste elektrisch leitende Piste (11) und die dritte elektrisch leitende Piste (14) einer Zelle (20) mit dem elektronischen System verbunden sind, das dazu ausgelegt ist, eine Änderung der elektrischen Kapazität des zweiten kapazitiven Sensors (30) für Scherung in der zweiten Richtung (X) zu messen, wobei das elektronische System dazu ausgelegt ist, daraus die Amplitude und die Richtung einer auf den kapazitiven Sensor (30) für Scherung entlang der zweiten Richtung (X) ausgeübten Scherkraft abzuleiten.

**6.** System aus einem Netzwerk aus Druck- und Scherbelastungssensoren gemäß Anspruch 5, bei dem die erste elektrisch leitende Piste (11) und die vierte elektrisch leitende Piste (16) einer Zelle (20) mit dem elektronischen System verbunden sind, das dazu ausgelegt ist, eine Änderung der elektrischen Kapazität des dritten kapazitiven Sensors (50) für Scherung in der dritten Richtung (Y) zu messen, wobei das elektronische System dazu ausgelegt ist, daraus die Amplitude und die Richtung einer auf den dritten kapazitiven Sensor (50) für Scherung entlang der dritten Richtung (Y) ausgeübten Scherkraft abzuleiten.

**7.** System aus einem Netzwerk aus Druck- und Scherbelastungssensoren gemäß einem der Ansprüche 1 bis 6, bei dem die elektrisch leitenden Pisten (11, 12, 14, 16) durch Drahtverbindungen oder drahtlos mit Mitteln zum Messen einer Änderung der elektrischen Kapazität der kapazitiven Sensoren (10, 30, 50) verbunden sind.

**8.** System aus einem Netzwerk aus Druck- und Scherbelastungssensoren gemäß einem der Ansprüche 1 bis 7 mit einer Vorrichtung für die Anzeige der Messungen der Druck- und/oder Scherkräfte, bei dem die Anzeigevorrichtung dazu ausgelegt ist, in Abhängigkeit von der Anordnung des Netzwerks aus Sensoren den durch jede Zelle des Netzwerks aus Sensoren gemessenen senkrechten Druck und/oder gleichzeitig die Amplitude und die Richtung der durch jede Zelle des Netzwerks aus Sensoren gemessenen Scherkraft grafisch darzustellen.

**9.** Sohle für Schuhe, die ein System aus einem Netzwerk aus Druck- und Scherbelastungssensoren gemäß einem der Ansprüche 1 bis 8 aufweist.

**Claims**

**1.** A pressure and shear sensor network system, comprising:

- a sheet of dielectric material (7) elastically deformable in compression and shear, the sheet of dielectric material (7) having a first face and a second face,

**characterized in that** the sensor network system includes:

- a network (100) comprising a plurality of pressure sensor cells (20), the cells (20) being arranged in at least three rows and at least three columns, each cell (20) comprising a first capacitive sensor (10) for sensing normal pressure in a first direction (Z), and wherein at least one cell of the sensor network comprises a second capacitive sensor (30) for sensing shear in a second direction (X) and a third capacitive sensor (50) for sensing shear in a third direction (Y),
- each capacitive sensor (10, 30, 50) being consisted of a first electrode (1, 3, 5) fixed to the first face of the sheet of dielectric material (7) and a second electrode (2, 4, 6) fixed to the second face of the sheet of dielectric material (7),
- said first electrodes (1, 3, 5) of the capacitive sensors (10, 30, 50) of a cell (20) being connected in series to a first electrically conductive track (11) connecting a row of cells of the sensor network;
- the second electrode (2) of the capacitive normal-pressure sensor (10) of a cell (20) being connected to a second electrically conductive track (12) connecting a column of capacitive normal-pressure sensors (10) of the sensor network, the second electrode (4) of the capacitive sensor (30) for sensing shear in the second direction (X) being connected to a third electrically conductive track (14) connecting a row of capacitive sensors (30) for sensing shear in the second direction (X) of the sensor network; and the second electrode (6) of the capacitive sensor (50) for sensing shear in the third direction (Y) being connected to a fourth electrically conductive track (16) connecting a column of capacitive sensors (50) for sensing shear in the third direction (Y) of the sensor network, said first and second electrodes (3, 4, 5, 6) of the capacitive shear sensors (30, 50) being comb-shaped,

and **in that** the sensor network system comprises addressing means adapted to measure the electrical capacitance of a capacitive sensor (10, 30, 50) located at the intersection of a row and a column, said row corresponding to a first track (11) connected to said first electrode (1, 3, 5) and said column corresponding to another track (12, 14, 16) connected to one of said second electrodes (2, 4, 6).

2. The pressure and shear sensor network system according to claim 1, wherein the sheet of dielectric material (7) elastically deformable in compression and shear is a material chosen among: a micro-architectured cork, an elastomer, a rubber, a urethane, a silicone, a butyl rubber, a polymer, a neoprene, a polyurethane, a polyisoprene or an urethane foam.

3. The pressure and shear sensor network system according to one of claims 1 to 2, wherein said first electrode (1, 3, 5) and said first electrically conductive track (11) are printed on a sheet (9) of electrically insulating and flexible material, and, respectively, wherein said second electrode (2, 4, 6) and said other electrically conductive tracks (12, 14, 16) are printed on another sheet (8) of electrically insulating and flexible material.

4. The pressure and shear sensor network system according to one of claims 1 to 3, wherein said first electrically conductive track (11) and the second electrically conductive track (12) of a cell (20) are connected to an electronic system adapted to measure a variation of the electrical capacitance of the capacitive normal-pressure sensor (10), the electronic system being adapted to deduce therefrom a normal pressure force applied to said capacitive normal-pressure sensor (10) along the first direction (Z).

5. The pressure sensor network system according to one of claims 1 to 4, wherein the first electrically conductive track (11) and the third electrically conductive track (14) of a cell (20) are connected to said electronic system, which is adapted to measure a variation of the electrical capacitance of the second capacitive sensor (30) for sensing shear in the second direction (X), the electronic system being adapted to deduce therefrom the amplitude and direction of a shear force applied to said capacitive sensor (20) for sensing shear along the second direction (X).

6. The pressure sensor network system according to claim 5, wherein the first electrically conductive track (11) and the fourth electrically conductive track (16) of a cell (20) are connected to said electronic system, which is adapted to measure a variation of the electrical capacitance of the third capacitive sensor (50) for sensing shear in the third direction (Y), the electronic system being adapted to deduce therefrom the amplitude and direction of a shear force applied to said third capacitive shear sensor (50) along the third direction (Y).

7. The pressure sensor network system according to one of claims 1 to 6, wherein said electrically conductive tracks (11, 12, 14, 16) are connected to means for measuring a variation of electrical capacitance of the capacitive sensors (10, 30, 50) by wired or wireless links.

8. The pressure sensor network system according to one of claims 1 to 7, comprising a device for displaying pressure and/or shear force measurements, wherein the display device is configured to represent graphically, as a function of the arrangement of the sensor network, the normal pressure measured by each cell of the sensor network and/or simultaneously the amplitude and direction of the shear force measured by each cell of the sensor network.

9. A shoe sole comprising a pressure sensor network system according to one of claims 1 to 8.

# Fig.1

# Fig.2

# Fig.3

# Fig.4

# Fig.5

Fig.6

# Fig.7

# Fig.8

# Fig.9

# Fig.10

$P_{MAX}(kPa)$

0    200    400

# Fig.11

$P_{MAX}(kPa)$

0    200    400

# Fig. 12

# Fig. 13

## RÉFÉRENCES CITÉES DANS LA DESCRIPTION

**Documents brevets cités dans la description**

- EP 20607876 A
- US 7343813 B
- US 2014076066 A
- US 2006247140 A
- FR 2878956
- US 5449002 A
- US 20130093437 A
- US 8250926 B

**Littérature non-brevet citée dans la description**

- **R. SUPRANENI ; Q. GUO ; Y. XIE ; D.J. YOUNG ; C.H. MASTRANGELO.** A three-axis high-resolution capacitive tactile imager system based on floating comb electrodes. *Journal of Micromechanis and Microengineering,* 2013, vol. 23, 075004